(19) Europäisches Patentamt

European Patent Office

Office européen des brevets

(11) **EP 1 734 115 A1**

(12) **EUROPEAN PATENT APPLICATION**
published in accordance with Art. 158(3) EPC

(43) Date of publication:
20.12.2006 Bulletin 2006/51

(51) Int Cl.:
*C12N 9/20* (2006.01)    *C12P 7/64* (2006.01)

(21) Application number: 05728498.6

(22) Date of filing: 08.04.2005

(86) International application number:
PCT/JP2005/006909

(87) International publication number:
WO 2005/097985 (20.10.2005 Gazette 2005/42)

(84) Designated Contracting States:
AT BE BG CH CY CZ DE DK EE ES FI FR GB GR HU IE IS IT LI LT LU MC NL PL PT RO SE SI SK TR

(30) Priority: 08.04.2004 JP 2004114444

(71) Applicant: The Nisshin OilliO Group, Ltd.
Tokyo 104-8285 (JP)

(72) Inventors:
• Suzuki, Junko,
The Nisshin OilliO Group, Ltd.
Yokosuka-shi, Kanagawa 2390832 (JP)
• Negishi, Satoshi,
The Nisshin OilliO Group, Ltd.
Yokosuka-shi,
Kanagawa 2390832 (JP)
• Arai, Yuri,
The Nisshin OilliO Group, Ltd.
Yokosuka-shi, Kanagawa 2390832 (JP)

• Sakurai, Chika,
The Nisshin OilliO Group, Ltd.
Yokosuka-shi, Kanagawa 2390832 (JP)
• Hirose, Tadashiro,
The Nisshin OilliO Group, Ltd.
Yokosuka-shi, Kanagawa 2390832 (JP)
• Uehara, Hidetaka,
The Nisshin OilliO Group, Ltd.
Yokosuka-shi, Kanagawa 2390832 (JP)
• Arimoto, Shin,
The Nisshin OilliO Group, Ltd.
Yokosuka-shi, Kanagawa 2390832 (JP)
• Suganuma, Tomomi,
The Nisshin OilliO Group, Ltd.
Yokosuka-shi, Kanagawa 2390832 (JP)

(74) Representative: Zimmermann, Gerd Heinrich
Zimmermann & Partner,
P.O. Box 330 920
80069 München (DE)

(54) **1,3-SPECIFIC LIPASE POWDER, METHOD OF PRODUCING THE SAME AND UTILIZATION OF THE SAME**

(57)    A method for producing a lipase powder, which comprises the step of spray drying an aqueous solution containing a lipase adjusted to pH 6 to 7.5; the lipase powder which is a 1,3-specific lipase derived from *Rhizomucor miehei,* spherical, and contains not more than 10% by weight of moisture; and a lipase composition wherein the lipase powder is immersed or impregnated in fatty oil. According to the present invention, a lipase powder of which 1,3-selectivity is improved can be provided.

EP 1 734 115 A1

**Description**

Technical Field of the Invention

The present invention relates to a 1,3-specific lipase powder (powdered lipase) which can be appropriately used in the reactions such as trans-esterification of various fatty oils (fat and oil); methods for producing the same; a lipase composition wherein the 1,3-specific lipase powder is immersed or impregnated (or soaked) in fatty oil; a trans-esterification method of fatty oil, which comprises the step of using the lipase powder, and the like.

Background of the Invention

Lipases are widely used in the reactions such as esterification of various carboxylic acids such as fatty acids with alcohols such as mono-alcohol and polyalcohol, and trans-esterification between plural carboxylates. In these, the trans-esterification method is an important technology not only as reforming animal and plant fatty oils but also as methods for producing various fatty esters, sugar esters and steroids. When a lipase, which is a fatty acid hydrolytic enzyme, is used as a catalyst of the above reactions, esterification can be conducted under the mild condition such as at room temperature to about 70°C. Therefore, the lipase can better inhibit side reactions and reduce energy costs compared with the existing chemical reactions. Besides those, a lipase as a catalyst is a natural product and, therefore, safe and secure. Further, the lipase can effectively produce the intended compounds through its substrate specificity and site specificity. However, even if lipase powder is used in esterification as itself, activity does not fully express. Further, it is difficult to uniformly disperse a lipase, which is basically a water-soluble product, into oily raw materials, and recover thereof is also difficult. Therefore, in the conventional methods, it is common to immobilize a lipase to some carriers, such as anion-exchange resin (Patent Literature 1), phenol adsorption resin (Patent Literature 2), a hydrophobic carrier (Patent Literature 3), cation-exchange resin (Patent Literature 4) and chelate resin (Patent Literature 5) and to use it in the reactions such as esterification and trans-esterification.

As mentioned above, a lipase has been conventionally immobilized and used in the esterification. However, the immobilized lipase loses an original lipase activity through the immobilization. In addition, when a porous carrier was used, the raw materials and products have gotten stuck in fme pores and, as a result, decreased the ester exchange ratio. Further, in the trans-esterification wherein the conventional immobilized lipase is used, water which a carrier retains is brought into the reaction system, and therefore, it has been difficult to prevent the side reactions such as production of diglyceride and monoglyceride in the trans-esterification of fatty oils.

In light of the situations mentioned above, various technologies have been developed wherein lipase powder is used. For example, a trans-esterification method is proposed wherein in the presence or absence of an inactive organic solvent(s), lipase powder is dispersed into a raw material(s) containing ester in the trans-esterification in such a manner that 90% or more of the particles of the dispersed lipase powder can keep particle size of 1 to 100μm in the reaction (Patent Literature 6). It is also proposed that enzyme powder is used, which is obtained by drying an enzyme solution (s) containing phospholipid and lipid-soluble vitamins (Patent Literature 7).

However, there has been desired a lipase powder wherein 1,3-selectivity of a 1,3-specific lipase is further improved.

[Patent Literature 1]    Japanese Patent Publication No. Sho 60-98984
[Patent Literature 2]    Japanese Patent Publication No. Sho 61-202688
[Patent Literature 3]    Japanese Patent Publication No. Hei 2-138986
[Patent Literature 4]    Japanese Patent Publication No. Hei 3-61485
[Patent Literature 5]    Japanese Patent Publication No. Hei 1-262795
[Patent Literature 6]    Japanese Patent No. 2668187
[Patent Literature 7]    Japanese Patent Publication No. 2000-106873

Disclosure of the Invention

An object of the present invention is to provide a lipase powder wherein 1,3-selectivity of a 1,3-specific lipase is improved.

Another object of the present invention is to provide lipase compositions wherein the lipase powder is immersed or impregnated in fatty oil.

A still another object of the present invention is to provide methods for producing the lipase powder.

A further object of the present invention is to a trans-esterification method of fatty oil, which comprises the step of using the lipase powder.

The above objects and other objects will be apparent from the following descriptions.

A 1,3-specific lipase derived from *Rhizomucor miehei* has extremely high 1,3-selectivity and is used in a manner that it is immobilized to anion-exchange resin. However, when the lipase is made in powder form and spherical, and contains not more than 10% by weight of moisture without using a carrier for immobilization, 1,3-selectivity thereof is further improved. The present invention has been completed on the basis of this finding.

Namely, the present invention provides a lipase powder which is a 1,3-specific lipase derived from *Rhizomucor miehei,* spherical, and contains not more than 10% by weight of moisture.

The present invention also provides a lipase composition wherein the lipase powder is immersed or impregnated in fatty oil.

The present invention further provides a method for producing the lipase powder, which comprises the step of spray drying an aqueous solution containing a lipase adjusted to pH 6 to 7.5.

The present invention further provides a lipase for trans-esterification containing the lipase powder.

The present invention further provides a trans-esterification method of fatty oil, which comprises the step of using the lipase for the trans-esterification.

Best Mode for Carrying out the Invention

The 1,3-specific lipase, which is the subject of the present invention, is a 1,3-specific lipase derived from *Rhizomucor miehei* belonging to *Rhizomucor* sp. However, *Rhizomucor miehei* sometimes used to belong to *Mucor* sp.

In the present invention, this lipase is used as a powder which is spherical and contains not more than 10% by weight of moisture.

The water content thereof is not more than 10%, and 6.5 to 8.5% by weight is preferable.

Although particle size of the lipase powder of the present invention can be optional, it is preferable that 90% by weight or more of the lipase powder has the particle size of 1 to 100$\mu$m. In this connection, it is preferable that an average particle size thereof be 20 to 80 $\mu$m, more preferably 20 to 50 $\mu$m.

The particle size of the lipase powder can be determined by, for example, Particle Size Distribution Analyzer (LA-500) of HORIBA, Ltd.

The above mentioned lipase powder can be easily prepared by spray drying an aqueous solution containing a lipase.

Here, examples of the aqueous solution containing a lipase include a lipase culture solution from which a cell body is removed, a purified culture solution thereof; a solution in which the lipase powder obtained from these culture solutions is dissolved and dispersed again; a solution in which the commercially available lipase powder is dissolved and dispersed again; and commercially available liquid lipase. In order to enhance lipase activity, it is more preferable that low-molecular-weight components such as salts are removed from the solution. In order to enhance the powder property, it is more preferable that low-molecular-weight components such as sugar are removed from the solution.

A lipase culture solution includes, for example, aqueous solutions containing soybean flour, peptone, corn steep liquor, K2HPO4, (NH4)2SO4, MgSO4 / 7H2O and the like. The concentrations thereof are as follows: the soybean flour is 0.1 to 20% by weight and preferably 1.0 to 10% by weight; peptone is 0.1 to 30% by weight and preferably 0.5 to 10% by weight; the corn steep liquor is 0.1 to 30% by weight and preferably 0.5 to 10% by weight; $K_2HPO_4$ is 0.01 to 20% by weight and preferably 0.1 to 5% by weight; $(NH_4)_2SO_4$ is 0.01 to 20% by weight and preferably 0.05 to 5% by weight; and $MgSO_4$ / $7H_2O$ is 0.01 to 20% by weight and preferably 0.05 to 5% by weight. The culture conditions thereof should be controlled as follows: the culture temperature is 10 to 40°C and preferably 20 to 35°C; the quantity of airflow is 0.1 to 2.0VVM and preferably 0.1 to 1.5VVM; the rotation speed for stirring is 100 to 800rpm and preferably 200 to 400rpm; pH is 3.0 to 10.0 and preferably 4.0 to 9.5.

The separation of a cell body is preferably conducted by centrifugation, the membrane filter procedure and the like. The removal of the low-molecular-weight components such as salts and sugar can be treated with ultrafiltration membranes. Specifically, after the treatment with ultrafiltration membranes, the aqueous solution containing a lipase is concentrated so as to become 1/2 volume thereof; and then, the same amount of a phosphate buffer as that of the concentrated solution is added thereto. By repeating these procedures once to 5 times, the aqueous solution containing a lipase can be obtained, from which the low-molecular-weight components are removed.

The centrifugation is preferably controlled to 200 to 20,000 x g. The pressure applied to the membrane filter is preferably controlled by microfiltration membranes, the filter press and the like to become not more than 3.0kg/m2. In case of enzymes in the cell body, it is preferable that cell breakage thereof is conducted by the homogenizer, Waring blender, the ultrasonic disruption, the French press, the ball mill and the like; then the cell residues are removed by centrifugation, the membrane filter procedure and the like. The rotation speed of the homogenizer for stirring is 500 to 30,000rpm and preferably 1,000 to 15,000rpm. The rotation speed of Waring blender is 500 to 10,000rpm and preferably 1,000 to 5,000rpm. The time for stirring is 0.5 to 10 minutes and preferably 1 to 5 minutes. It is preferable that the ultrasonic disruption is conducted under the condition of 1 to 50 KHz and more preferably 10 to 20 KHz. It is preferable that the ball mill has glass pellets having the diameter of 0.1 to 0.5mm.

In the present invention, it is preferably that the aqueous solution containing a lipase is that containing 5 to 30% by weight of lipase as a solid content.

Here, the concentration of the solid content in the aqueous solution containing a lipase can be determined as Brix.% by using, for example, the sugar content analyzer (Refractormeter)(CIS Corporation.: BRX-242).

Immediately before the drying process such as spray drying, it is preferable that the aqueous solution containing a lipase is adjusted to pH 6 to 7.5. It is more preferable that it is adjusted to pH 7 or lower and further more preferably to pH 6.5 to 7.0. The pH adjustment can be conducted in any stage before the drying process such as spray drying. The pH of the aqueous solution containing a lipase may be preliminarily adjusted so that pH immediately before the drying process is within the above range. Various alkaline chemicals and acids can be used for the adjustment of pH and alkali metal hydroxides such as sodium hydroxide are preferably used.

In some stage before the drying process, the aqueous solution containing a lipase may be concentrated. The concentration methods are not particularly limited and they include evaporator, flash evaporator, the concentration by ultrafiltration, the concentration by microfiltration, salting out by inorganic salts, precipitation methods with solvents, absorption methods with ion-exchange cellulose and the like, and water absorption methods with water-absorbing gels. Among these, the concentration by ultrafiltration and evaporator are preferable. The module for the concentration by ultrafiltration is preferably a flat membrane or a hollow fiber membrane having a fractioned molecular weight of 3,000 to 100,000 and more preferably 6,000 to 50,000. The materials of the membrane are preferably polyacrylonitrile, polysulfonic and the like.

It is preferable that spray drying is conducted by spray dryers such as nozzle countercurrent flow, disk countercurrent flow, nozzle concurrent flow and disk concurrent flow, and the disk concurrent flow is more preferable. The spray drying is preferably controlled as follows: the rotation speed of the atomizer is 4,000 to 20,000rpm; and heating is 100 to 200°C for inlet temperature and 40 to 100°C for outlet temperature.

The lipase powder thus prepared can be used as itself. However, it is preferable, from the point of handling, that it is used as a lipase composition wherein the lipase powder is immersed or impregnated in fatty oil. Here, the mass of the fatty oil in the lipase composition is preferably 0.1 to 20 times and more preferably 1 to 20 times as heavy as the lipase powder.

The lipase composition can be easily obtained by adding the fatty oil to the lipase powder produced by spray drying and the like; and then uniformly stirring the mixture by a stirrer, three-one motor, and the like. It can also be easily obtained by preliminarily adding the fatty oil to a powder recovering region of a spray dryer; uniformly stirring the mixture after the recovering; and then removing the excess fatty oil by filtration.

The fatty oils for immersing or infiltrating the lipase powder are not particularly limited. They include vegetable oils such as canola oil, soybean oil, higholeic sunflower oil, olive oil, safflower oil, corn oil, palm oil and sesame-seed oil; triacylglycerols such as triolein (glycerol trioleate), tricaprilyn (glycerol trioctanoate), triacetin (glycerol triacetate)and tributyrin (glycerol tributyrate); and the mixture of one or more thereof such as fatty ester and sterol ester.

The trans-esterification of the fatty oil and the like can be effectively conducted by the ordinary method using the lipase powder.

According to the present invention, 1,3-selectivity of a 1,3-specific lipase is extremely improved, and the fatty acid residue which is located on the second position of triglyceride as a raw material can be retained in the trans-esterification manufacture at an extremely high percentage.

The following Examples will further illustrate the present invention in detail.

Example 1

The low-molecular-weight components were removed by using the UF module (ASAHI KASEI CHEMICALS CORPORATION): SIP-0013) from a lipase derived from *Rhizomucor miehei* of Novozymes Japan Ltd. (Trade name: Palatase 20000L), which was dissolved and dispersed in an aqueous solution to obtain an aqueous solution containing a lipase (the concentration of the solid content: 10.6% by weight). Specifically, liquid lipase (Palatase 20000L) was treated with ultrafiltration module under cooling with ice and concentrated so as to become 1/2 volume thereof. Then, the same amount of a 0.01M phosphate buffer as that of the concentrated solution was added thereto. The same procedures of ultrafiltration and the addition of a phosphate buffer were conducted three times to the obtained solution to obtain an aqueous solution containing a lipase (the volume ratio: a lipase concentrated solution / buffer = 1/1).

The pH of the aqueous solution containing a lipase was adjusted with an aqueous solution of sodium hydroxide to become the pH 6.8 to 6.9.

Then, the solution was sprayed by using a spray dryer (SD-1000: TOKYO RIKAKIKAI CO, LTD) under the conditions of inlet temperature: 130°C, the air content for drying: 0.7 to 1.1m$^3$/min, and spray pressure: 11 to 12kpa to obtain lipase powder. The shape of the thus-obtained lipase powder was spherical, 90% by weight or more of the lipase powder has a particle size of 1 to 100$\mu$m and the average particle size thereof was 8.2$\mu$m. The particle size was determined by Particle Size Distribution Analyzer (LA-500) of HORIBA, Ltd. The water content after the dry heat at 105°C for 1 hour was 7.9% by weight.

The concentration of the solid content in the aqueous solution containing a lipase was determined as Brix.% by using the sugar content analyzer (Refractormeter) (CIS Corporation.: BRX-242).

Comparative Example 1

Herein used was Lipozyme RM IM of Novozymes Japan Ltd. (wherein a lipase derived from *Rhizomucor miehei* was immobilized to anion-exchange resin.

Comparative Example 2

The same procedure was conducted as that of Example 1 except that freeze-drying was conducted instead of spray drying to obtain lipase powder. The freeze-drying was conducted as follows. An aqueous solution containing a lipase whose pH was adjusted to 6.8 to 6.9 was poured into a recovery flask and frozen by dry ice methanol. Then, the frozen material was freeze-dried by using a freeze-dryer (FDU-830) of TOKYO RIKAKIKAI CO, LTD at 0.15Torr for 1 day. After drying, the resultant was lightly crushed in a mortar to obtain lipase powder. The shape of the thus-obtained lipase powder was amorphous, and the water content thereof was 11.3% by weight.

1,3-selectivity of each Example 1, Comparative Example 1 and Comparative Example 2 was determined by the following method. The measurements of 1,3-selectivity of Example 1 and Comparative Example 1 were indicated in Table 1. Regarding the lipase powder obtained in Comparative Example 2, there was not any trans-esterification activity at all.

Determination of 1,3-selectivity

1 mol of GRYCERYL-1,3-PALMITATE-2-OLEATE(POP) and 3 mol of OCTANOIC ETHYL(C8Et) were used as reaction substrates. Lipase powder was added thereto in such that the enzymatic activities become 0.5 to 5w% of the substrates. The reaction was conducted at 60°C and samples thereof were taken with lapse of time and diluted with hexane. The GC analysis was conducted to the samples, and the reaction rates of the 1.3 position (C16:0Et) and the second position (C18:1Et) were obtained by the following formulae.

$$C16{:}0Et(\%) = \{C16{:}0Et \text{ aria}/(C16Et + C18{:}1Et \text{ area} + C8Et \text{ area})\} \times 100$$

$$C18{:}1Et(\%) = \{C18{:}1Et \text{ area}/(C16Et + C18{:}1Et \text{ area} + C8Et \text{ area})\} \times 100$$

The reaction rate constant K was determined from the reaction rates of each samples at each time by using the analysis software (orijin ver.6.1). At this time, the value of the final reaction rate is changeable. The reactivity of the 1.3 position was calculated when the reactivity of the second position was regarded as 1.

[GC conditions]

| | |
|---|---|
| Column: | DB-1ht 5m |
| Injection rate: | 1μl |
| Carrier gas: | helium |
| Temperature in the vaporizing chamber: | 360°C |
| Temperature of the detector: | 370°C |
| Column temperature: | beginning 50°C, temperature rising 15°C/min, end 370°C |

Table 1

| | Condition | 1,3-selectivity | (improved rate) |
|---|---|---|---|
| Comp. Example 1 | (immobilized lipase) | 11.1 | |

| | (continued) | | |
| --- | --- | --- | --- |
| | Condition | 1,3-selectivity | (improved rate) |
| Example 1 | (spray drying) | 20.8 | (Δ 87%) |

From the results of Table 1, according to the present invention, it was learned that 1,3-specific lipase was improved by 87%.

Example 2

The 5 times amount of canola oil as that of lipase powder was added to the lipase powder obtained in Example 1, and the lipase powder was immersed and impregnated in the canola oil. The extra fatty oil was removed by filtration and a lipase composition was prepared wherein the weight of the lipase powder / the canola oil was 55/45.

**Claims**

1. A lipase powder which is a 1,3-specific lipase derived from *Rhizomucor miehei,* spherical, and contains not more than 10% by weight of moisture.

2. The lipase powder according to claim 1 which is produced by spray drying an aqueous solution containing a lipase adjusted to pH 6 to 7.5.

3. The lipase powder according to claim 2 which is produced by spray drying an aqueous solution containing a lipase adjusted to pH 6.5 to 7.

4. The lipase powder according to claims 2 or 3, wherein the aqueous solution containing a lipase is a lipase culture solution from which a cell body is removed, or a purified culture solution thereof.

5. The lipase powder according to any of claims 2 to 4, wherein the aqueous solution containing a lipase is treated with ultrafiltration membranes.

6. The lipase powder according to any of claims 1 to 5, wherein 90% by weight or more of the lipase powder has a particle size of 1 to 100μm.

7. The lipase powder according to any of claims 1 to 6 which has a moisture content of 6.5 to 8.5 % by weight.

8. A lipase composition wherein the lipase powder according to any of claims 1 to 7 is immersed or impregnated in fatty oil.

9. The lipase composition according to claim 8, wherein the mass of the fatty oil in the lipase composition is 0.1 to 20 times the lipase powder.

10. A method for producing the lipase powder according to any of claims 1 to 7, which comprises the step of spray drying an aqueous solution containing a lipase adjusted to pH 6 to 7.5.

11. A lipase for trans-esterification containing the lipase powder according to any of claims 1 to 7.

12. A trans-esterification method of fatty oil, which comprises the step of using the lipase for the trans-esterification according to claim 11.

## INTERNATIONAL SEARCH REPORT

| | International application No. |
|---|---|
| | PCT/JP2005/006909 |

| A. CLASSIFICATION OF SUBJECT MATTER |
|---|
| Int.Cl$^7$  C12N9/20, C12P7/64 |

According to International Patent Classification (IPC) or to both national classification and IPC

| B. FIELDS SEARCHED |
|---|
| Minimum documentation searched (classification system followed by classification symbols)<br>    Int.Cl$^7$  C12N9/20, C12P7/64 |
| Documentation searched other than minimum documentation to the extent that such documents are included in the fields searched |
| Electronic data base consulted during the international search (name of data base and, where practicable, search terms used)<br>    BIOSIS/WPI(DIALOG), JSTPlus(JOIS) |

C. DOCUMENTS CONSIDERED TO BE RELEVANT

| Category* | Citation of document, with indication, where appropriate, of the relevant passages | Relevant to claim No. |
|---|---|---|
| X<br>Y | Decagny B. et al., 1H-NMR on line monitoring of water activity during lipase catalysed esterification, Biochim.Biophys.Acta., 1998, Vol.1387, Nos.1 to 2, pages 129 to 135 | 1-9,11-12<br>10 |
| Y | JP 07-079789 A  (The Nisshin Oil Mills, Ltd.),<br>28 March, 1995 (28.03.95),<br>Full text<br>& US 5480787 A | 1-12 |
| Y | JP 54-017179 A  (Sankyo Co., Ltd.),<br>08 February, 1979 (08.02.79),<br>Full text<br>(Family: none) | 1-12 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    07 July, 2005 (07.07.05) | Date of mailing of the international search report<br>    09 August, 2005 (09.08.05) |
|---|---|
| Name and mailing address of the ISA/<br>    Japanese Patent Office | Authorized officer |
| Facsimile No. | Telephone No. |

Form PCT/ISA/210 (second sheet) (January 2004)

**INTERNATIONAL SEARCH REPORT**

International application No.

PCT/JP2005/0006909

C (Continuation).  DOCUMENTS CONSIDERED TO BE RELEVANT

| Category* | Citation of document, with indication, where appropriate, of the relevant passages | Relevant to claim No. |
|---|---|---|
| Y | Xiao Y.W. et al., Purification and partial characterization of Rhizomucor miehei lipase for ester synthesis, Appl.Biochem.Biotech., 1996, Vol.59, No.2, pages 145 to 158 | 1-12 |

Form PCT/ISA/210 (continuation of second sheet) (January 2004)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 60098984 A
- JP 61202688 A
- JP 2138986 A
- JP 3061485 A
- JP 1262795 A
- JP 2668187 B
- JP 2000106873 A